Europäisches Patentamt

European Patent Office    (11) Publication number: **0 064 337**

Office européen des brevets    **B1**

(12)    **EUROPEAN PATENT SPECIFICATION**

(45) Date of publication of patent specification: **12.02.86**    (51) Int. Cl.⁴: **G 01 N 27/56,** G 01 N 27/30

(21) Application number: **82301879.1**

(22) Date of filing: **08.04.82**

(54) Carbon dioxide measurement.

(30) Priority: **30.04.81 GB 8113365**

(43) Date of publication of application:
**10.11.82 Bulletin 82/45**

(45) Publication of the grant of the patent:
**12.02.86 Bulletin 86/07**

(84) Designated Contracting States:
**CH DE FR IT LI**

(56) References cited:
DE-A-2 832 501
US-A-3 855 096
US-A-3 900 382
US-A-3 957 613

JOURNAL OF ELECTROANALYTICAL
CHEMISTRY, Vol. 9, 1965, California, USA J.L.
ROBERTS, JR. et al. "Voltametric
Determination of Carbon Dioxide Using
Dimethylsulfoxide as a Solvent" pages 1 to 7

The file contains technical information
submitted after the application was filed and
not included in this specification

(73) Proprietor: **NATIONAL RESEARCH
DEVELOPMENT CORPORATION
101 Newington Causeway
London SE1 6BU (GB)**

(72) Inventor: **Albery, Wyndham John
Flat 8 53 Queens Gardens
London W2 (GB)**

(74) Representative: **Percy, Richard Keith
Patent Department National Research
Development Corporation 101 Newington
Causeway
London SE1 6BU (GB)**

(56) References cited:
ANALYTICAL CHEMISTRY, Vol. 39, No. 3, 1967,
California, USA L.V. HAYNES et al.
"Electrochemistry of Carbon Dioxide in
Dimenthyl Sulfoxide at Gold and Mercury
Electrodes" pages 332-338

Courier Press, Leamington Spa, England.

# Description

This invention relates to the electrochemical measurement of carbon dioxide in a fluid which may contain oxygen.

It is known to sense carbon dioxide by electrochemical methods such as polarography. In J. Electroanal. Chem. *9*, 1965, pp 1 to 7 in a report by J. L. Roberts and D. T. Sawyer, and in Anal. Chem. *39* (3), 1967, pp 332 to 338, in a report by L. V. Haynes and D. T. Sawyer, the concentration of $CO_2$ is measured by reduction at a stationary electrode in dimethylsulphoxide, and analysis of the cyclic voltammagram. However, it was essential to exclude oxygen, because oxygen is more easily reduced than $CO_2$. The almost universal copresence of oxygen with $CO_2$ in normal circumstances means that such a method is not generally applicable.

US—A—4,197,853 and the corresponding DE—A—2,832,501 describe a sensor for measurement of carbon dioxide in the presence of oxygen, comprising a polymeric membrane permeable to oxygen and carbon dioxide, arranged so that one surface thereof is exposed to the carbon dioxide — and oxygen containing fluid and its opposite surface is in contact with an aqueous alkaline electrolyte in a single chamber in which two electrodes are present, one being a pH electrode for measuring carbon dioxide and the other an electrode at which oxygen is reduced.

US—A—3,957,613 describes a device for measurement of carbon dioxide in the presence of oxygen, comprising separate sensors housed within the same probe. One sensor consists essentially of a membrane permeable to carbon dioxide and an aqueous bicarbonate electrolyte in which is disposed a pH electrode for measuring carbon dioxide. The other sensor consists essentially of an oxygen-permeable membrane and an aqueous alkali metal halide electrolyte in which is disposed an electrode at which oxygen is reduced.

In both the above patents carbon dioxide is measured potentiometrically, i.e. the potential difference between the working and reference electrodes is measured. This depends on the pH of the electrolyte, which depends on the concentration of carbon dioxide. A problem with these methods is that they are easily affected by small changes in pH of the electrolyte due to factors other than change in concentration of carbon dioxide.

It has now been found possible to measure carbon dioxide in the presence of oxygen by a more direct method, which does not involve pH sensing, and which gives a rapid response time.

According to the invention, apparatus for sensing the presence of carbon dioxide in a fluid which may contain oxygen includes:

a first layer of high polymeric material which is permeable to oxygen and is arranged so that one surface thereof is exposed to the fluid and the other surface is in communication with a layer of an aqueous first electrolyte and with a first working electrode;

a second layer (22) of high polymeric material which is permeable to carbon dioxide and arranged so that one surface thereof is exposed to the fluid and the other surface thereof is in contact with a layer of a second electrolyte, which is in contact with a second working electrode (16); and

circuit means arranged to hold the first working electrode at a potential at which the oxygen is reduced, and including first and second counter electrodes (28, 30) in contact with the respective electrolytes;

characterised in that:

the first layer (32) of high polymeric material is permeable to both carbon dioxide and oxygen and has in contact with its said other surface a porous layer of metal (34) which constitutes the first working electrode;

the second layer (22) of high polymeric material is arranged "in series" with the first layer (32), so that the one surface of the second layer is exposed to carbon dioxide from which oxygen has been removed by passage through the first electrolyte;

the second electrolyte is non-aqueous; and

the circuit means (48) is arranged to hold the first working electrode at a potential such that oxygen but not carbon dioxide is reduced in the aqueous first electrolyte, to hold the second working electrode at a potential such that carbon dioxide is reduced in the non-aqueous second electrolyte, and to sense any current flowing between the second working and second counter electrodes.

The invention also includes a method of sensing the presence of carbon dioxide in a fluid which may contain oxygen, including:

arranging a first layer of high polymeric material which is permeable to oxygen, so that one surface thereof is exposed to the fluid and the other surface is in communication with a layer of an aqueous first electrolyte and with a first working electrode;

arranging a second layer (22) of high polymeric material which is permeable to carbon dioxide, so that one surface thereof is exposed to the fluid and the other surface thereof is in contact with a second working electrode (16); and

holding the first working electrode at a potential at which the oxygen is reduced;

characterised in that:

the first layer (32) of high polymeric material is permeable to both carbon dioxide and oxygen and has in contact with its said other surface a porous layer of metal (34) which constitutes the first working electrode;

the second layer (22) of high polymeric material is arranged "in series" with the first layer (32), so that one surface of the second layer is exposed to carbon dioxide from which oxygen has been removed by passage through the first electrolyte;

the second electrolyte is non-aqueous; and

the first working electrode is held at a potential such that oxygen but not carbon dioxide is reduced in the aqueous first electrolyte, the second working electrode is held at a potential such that carbon dioxide is reduced in the non-aqueous second electrolyte, and any current flowing through the second working electrode is sensed.

The first layer and first working electrode may together be in the form of a metallised membrane of a high polymeric material. Metallised membranes are known in themselves, for example from UA—A—3,855,096. The non-aqueous electrolyte may be dimethylsulphoxide (DMSO).

Usually the potentials of the first and second working electrodes are held constant with respect to first and second reference electrodes in contact with the aqueous electrolyte and the DMSO respectively. The currents generated in the aqueous electrolyte by reduction of any oxygen present and in the DMSO by reduction of any carbon dioxide present flow through first and second counter electrodes in contact with the respective electrolytes.

Preferably the first working electrode is of larger area than the second working electrode, and the diffusion of the gases in the fluid under test is restricted to ensure that all of the oxygen passing through the first polymeric layer to the first working electrode is reduced at that electrode. The restriction may be in the form of an aperture of area less than the area of the first working electrode.

The invention will now be described by way of example only with reference to the accompanying drawings in which:—

Figure 1 is a gas sensor according to the invention shown partly in longitudinal section and partly exploded; and

Figure 2 is a plan view of the sensor electrodes.

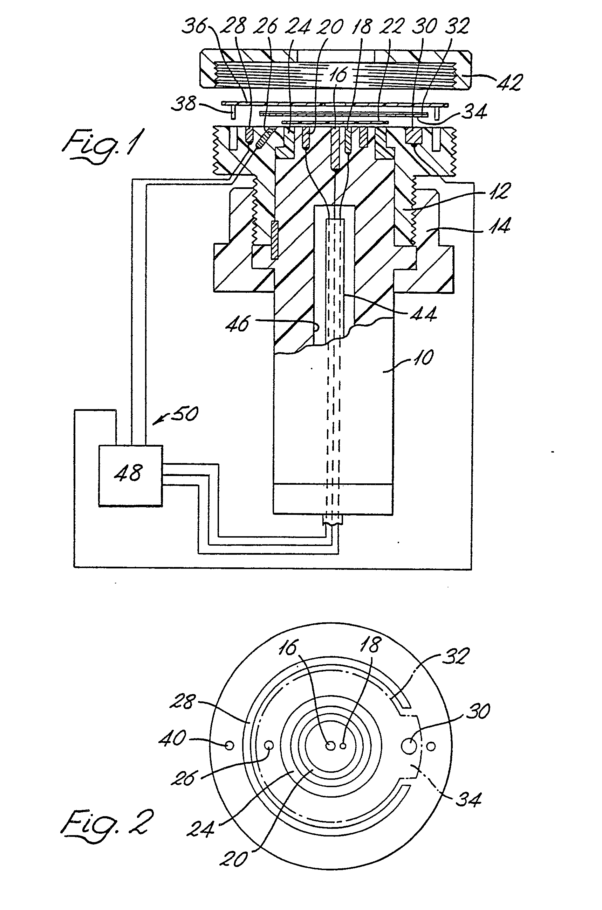

In Figure 1, a sensor body has an inner part 10 and an outer part 12 held together by a nut 14. The upper faces of both the inner and outer parts are coplanar, and six electrodes or electrode contacts are recessed to lie flush with the coplanar surfaces.

Referring to both Figures 1 and 2, in the inner part 10 are a central second working electrode 16 in the form of a gold or silver rod, and an adjacent silver/silver chloride reference electrode 18 both surrounded by a silver counter electrode 20. A circular membrane 22 of high polymeric material covers the electrodes 16, 18, 20 and is also supported by a washer 24 around the outside of the inner body 10. Between the membrane 22 and the electrodes is a thin layer (not illustrated) of dimethylsulphoxide.

The coplanar surface of the outer body 12 carries a silver/silver chloride reference electrode 26 and a silver counter electrode 28 which is 'C' shaped. Between the arms of the 'C' there is a silver epoxy stud 30, and above the membrane 22 is a membrane 32, the lower surface of which is covered with a layer of sputtered gold 34, thus forming a metallised membrane electrode which, for convenience, is referred to in the following description as "membrane electrode 32". The gold layer is largely circular, of diameter smaller than the counter electrode 28, but with an extension coinciding with the aperture between the arms of electrode 28 to make contact with the stud 30.

Between the metallised membrane electrode 32 and the membrane 22 is a thin layer (not illustrated) of aqueous electrolyte.

Above the membrane electrode 32 is a circular washer 36 which has location pins 38 which can enter depressions 40 in the upper surface of the outer body 12. When the washer 36 is held in position by a screw cap 42, the metallised membrane 32 is pressed towards the membrane 22, part of the metallised surface of membrane 32 being held in contact with the stud 30, and the membrane 22 is pressed towards the washer 24. The thin layers of aqueous electrolyte and of DMSO remain between the membranes and the membrane 22 and body 10 respectively.

The screw cap has a central aperture, and the washer 36 has a smaller central aperture through both of which an atmosphere or fluid can pass to reach the upper, unmetallised surface of the membrane electrode 32.

Wire contacts to the electrodes 16, 18 and 20, which are involved in carbon dioxide sensing, pass through a cable 44 in a cylindrical aperture 46 within the inner body 10 to a measuring and control circuit 48. Wire contacts to the electrodes 26, 28 and electrode contact 30 pass through the outer body 12 to the circuit 48. The electrodes 26, 28 and the metallised membrane electrode 32 are the electrodes involved in oxygen removal. The circuit 48 is arranged to hold the first working electrode, i.e. membrane electrode 32, at a potential at which oxygen is reduced but carbon dioxide is not, and to hold the second working electrode 16 at a potential at which carbon dioxide is reduced, and to sense any current flowing between electrode 16 and the counter electrode 20. The potentials are held constant with respect to the respective reference electrodes 18, 26.

In operation, an atmosphere or fluid under test passes through the apertures in the screw cap 42 and washer 36 to the metallised membrane electrode 32. Oxygen and carbon dioxide gas pass through the membrane 32 and through the metallised layer to the aqueous electrolyte at which oxygen is reduced. The reduction current is proportional to the oxygen concentration and is measured by the circuit 48. Carbon dioxide gas passes through the aqueous electrolyte and through the membrane 22 to the DMSO layer in contact with the second working electrode 16 at which the $CO_2$ gas is reduced. This produces a current proportional to the $CO_2$ concentration, which is measured by the circuit 48.

Typically, the metallised membrane electrode 32 is a layer of 'Teflon' (Registered Trade Mark) 12 micrometers thick and covered with a sputtered layer of gold. The aqueous electrolyte is buffered to a pH of 5 with potassium hydrogen phthalate and the metal layer of membrane electrode is held at a potential of −0.6 Volts. The membrane 22 is typically a layer of 'Teflon' (Registered Trade Mark) 12 micrometres thick, and the second working electrode 16 is held at a potential of −2.3 Volts.

Alternatively, the metallised membrane electrode may be prepared by painting a porous membrane of 'Gorotex' (Registered Trade Mark) with a gold resinate solution and heating to leave a layer of gold.

As an alternative to silver, the second working electrode 16 may be made of mercury, nickel, gold, lead or glassy carbon.

A carbon dioxide sensor according to the invention may have a response time as fast as 5 seconds to a 90% concentration of carbon dioxide. Concentrations as low as 0.1% of $CO_2$ may be detected, and the sensor is suitable for use in medical applications, when both oxygen and carbon dioxide concentrations can be measured.

**Claims**

1. Apparatus for sensing the presence of carbon dioxide in a fluid which may contain oxygen, including:

a first layer of high polymeric material which is permeable to oxygen and is arranged so that one surface thereof is exposed to the fluid and the other surface is in communication with a layer of an aqueous first electrolyte and with a first working electrode;

a second layer (22) of high polymeric material which is permeable to carbon dioxide and arranged so that one surface thereof is exposed to the fluid and the other surface thereof is in contact with a layer of a second electrolyte, which is in contact with a second working electrode (16); and

circuit means arranged to hold the first working electrode at a potential at which the oxygen is reduced, and including first and second counter electrodes (28, 30) in contact with the respective electrolytes;

characterised in that:

the first layer (32) of high polymeric material is permeable to both carbon dioxide and oxygen and has in contact with its said other surface a porous layer of metal (34) which constitutes the first working electrode;

the second layer (22) of high polymeric material is arranged "in series" with the first layer (32), so that the one surface of the second layer is exposed to carbon dioxide from which oxygen has been removed by passage through the first electrolyte;

the second electrolyte is non-aqueous; and

the circuit means (48) is arranged to hold the first working electrode at a potential such that oxygen but not carbon dioxide is reduced in the aqueous first electrolyte, to hold the second working electrode at a potential such that carbon dioxide is reduced in the non-aqueous second electrolyte, and to sense any current flowing between the second working and second counter electrodes.

2. Apparatus according to Claim 1 characterised in that the first layer of high polymeric material (32) and the first working electrode (34) take the form of a metallised membrane of the high polymeric material.

3. Apparatus according to Claim 1 or Claim 2 in which the non-aqueous, second electrolyte is dimethylsulphoxide.

4. Apparatus according to any preceding claim characterised in that it also includes a first reference electrode (26) in contact with the first aqueous electrolyte; and a second reference electrode (18) in contact with the second, non-aqueous electrolyte; and in that the circuit means (48) includes means for maintaining the potential of each working electrode constant with respect to its respective reference electrode.

5. Apparatus according to any preceding claim in which the first working electrode is of larger area than the second working electrode (16), and there is further provided a restriction (42) to restrict access of the fluid to the first high polymeric layer (32).

6. A method of sensing the presence of carbon dioxide in a fluid which may contain oxygen, including:

arranging a first layer of high polymeric material which is permeable to oxygen, so that one surface thereof is exposed to the fluid and the other surface is in communication with a layer of an aqueous first electrolyte and with a first working electrode;

arranging a second layer (22) of high polymeric material which is permeable to carbon dioxide, so that one surface thereof is exposed to the fluid and the other surface thereof is in contact with a second working electrode (16); and

holding the first working electrode at a potential at which the oxygen is reduced;

characterised in that:

the first layer (32) of high polymeric material is permeable to both carbon dioxide and oxygen and has in contact with its said other surface a porous layer of metal (34) which constitutes the first working electrode;

the second layer (22) of high polymeric material is arranged "in series" with the first layer (32), so that one surface of the second layer is exposed to carbon dioxide from which oxygen has been removed by passage through the first electrolyte;

the second electrolyte is non-aqueous; and

the first working electrode is held at a potential such that oxygen but not carbon dioxide is reduced in the aqueous first electrolyte, and any current flowing through the second working electrode is sensed.

**Patentansprüche**

1. Vorrichtung zur Feststellung des Vorliegens von Kohlendioxid in einem fluiden Medium, das Sauerstoff enthalten kann, enthaltend:

eine erste Schicht aus hochpolymerem Material, die sauerstoffdurchlässig und so angeordnet ist, daß eine ihrer Oberflächen dem fluiden Medium ausgesetzt und die andere Oberfläche in Verbindung mit einer Schicht eines wässrigen ersten Elektrolyten und mit einer ersten Arbeitselektrode ist;

eine zweite Schicht (22) aus hochpolymerem Material, die kohlendioxiddurchlässig und so angeordnet ist, daß eine ihrer Oberflächen dem fluiden Medium ausgesetzt ist und die andere Oberfläche in Kontakt mit einer Schicht eines zweiten Elektrolyten steht, der in Kontakt mit einer zweiten Arbeitselektrode (16) ist;

und eine Schaltung, die so angeordnet ist, daß die erste Arbeitselektrode bei einem Potential gehalten wird, bei welchem Sauerstoff reduziert wird, und die erste und zweite Gegenelektroden (28, 30) in Kontakt mit den jeweiligen Elektrolyten umfaßt.

dadurch gekennzeichnet, daß die erste Schicht (32) aus hochpolymerem Material sowohl für Kohlendioxid als auch für Sauerstoff durchlässig ist und mit ihrer anderen Oberfläche in Kontakt mit einer porösen Schicht aus Metall (34) steht, welche die erste Arbeitselektrode darstellt;

daß die zweite Schicht (22) aus hochpolymerem Material "in Reihe" mit der ersten Schicht (32) angeordnet ist, so daß die eine Oberfläche der zweiten Schicht dem Kohlendioxid ausgesetzt ist, aus welchem Sauerstoff entfernt wurde, indem es durch den ersten Elektrolyt geleitet wurde;

daß der zweite Elektrolyt nicht wässrig ist;

und daß die Schaltung (48) so angeordnet ist, daß die ertse Arbeitselektrode auf einem solchen Potential gehalten wird, daß Sauerstoff, jedoch nicht Kohlendioxid im ersten wässrigen Elekrolyten reduziert wird, daß die zweite Arbeitselektrode auf einem solchen Potential gehalten wird, daß Kohlendioxid im nicht wässrigen zweiten Elektrolyten reduziert wird, und daß jeder Strom, der zwischen der zweiten Arbeits— und der zweiten Gegenelektrode fließt, festgestellt wird.

2. Vorrichtung nach Anspruch 1, dadurch gekennzeichnet, daß die erste Schicht aus hochpolymerem Material (32) und die erste Arbeitselektrode (34) die Form einer metallisierten Membran des hochpolymeren Materials haben.

3. Vorrichtung nach Anspruch 1 oder 2, dadurch gekennzeichnet, daß der nicht wässrige zweite Elektrolyt Dimethylsulfoxyd ist.

4. Vorrichtung nach einem der vorhergehenden Ansprüche, dadurch gekennzeichnet, daß sie auch eine erste Bezugselektrode (26) in Kontakt mit dem ersten wässrigen Elektrolyt und eine zweite Bezugselektrode (18) in Kontakt mit dem zweiten, nicht wässrigen Elektrolyt umfaßt, und daß die Schaltung (48) Mittel zur Konstanthaltung des Potentials jeder Arbeitselektrode bezüglich der jeweiligen Bezugselektrode umfaßt.

5. Vorrichtung nach einem der vorhergehenden Ansprüche, dadurch gekennzeichnet, daß die erste Arbeitselektrode eine größere Fläche hat als die zweite Arbeitselektrode (16), und daß weiter eine Verengung (42) vorgesehen ist, um den Zugang des fluiden Mediums zur ersten Hochpolymerschicht (32) zu begrenzen.

6. Verfahren zur Feststellung des Vorliegens von Kohlendioxid in einem fluiden Medium, das Sauerstoff enthalten kann, wobei man eine erste Schicht aus hochpolymerem Material, die sauerstoffdurchlässig ist, so anordnet, daß eine ihrer Oberflächen dem fluiden Medium ausgesetzt und die andere Oberfläche in Verbindung mit einer Schicht eines wässrigen ersten Elektrolyten und mit einer ersten Arbeitselektrode ist; wobei man

eine zweite Schicht (22) aus hochpolymerem Material, die kohlendioxiddurchlässig ist, so anordnet, daß eine iherer Oberflächen dem fluiden Medium ausgesetzt ist und die andere Oberfläche in Kontakt mit einer zweiten Arbeitselektrode (16) steht und wobei man

die erste Arbeitselektrode auf einem Potential hält, bei welchem Sauerstoff reduziert wird, dadurch gekennzeichnet, daß die erste Schicht (32) aus hochpolymerem Material sowohl für Kohlendioxid als auch für Sauerstoff durchlässig ist und mit ihrer anderen Oberfläche in Kontakt mit einer porösen Schicht aus Metall (34) steht, welche die erste Arbeitselektrode darstellt; daß man

die zweite Schicht (22) des hochpolymeren Materials "in Reihe" mit der ersten Schicht (32) anordnet, so daß die eine Oberfläche der zweiten Schicht dem Kohlendioxid ausgesetzt ist, aus welchem Sauerstoff entfernt wurde, indem es durch einen ersten Elektrolyt geleitet wurde;

daß der zweite Elektrolyt nicht wässrig ist;

daß man die erste Arbeitselektrode auf einem solchen Potential hält, daß Sauerstoff, jedoch nicht Kohlendioxid im ersten wässrigen Elektrolyten reduziert wird und daß jeder Strom, der durch die zweite Arbeitselektrode fließt, festgestellt wird.

## Revendications

1. Appareil pour détecter la présence du bioxyde de carbone dans un fluide pouvant contenir de l'oxygène, comprenant:

une première couche d'une matière qui est un haut polymère perméable à l'oxygène et est agencée de manière qu'une de ses surfaces soit exposée au fluide et que l'autre surface communique avec une couche d'un premier électrolyte aqueux et avec une première électrode de travail;

une seconde couche (22) de haut polymère qui est une matière perméable au bioxyde de carbone et qui est agencée de manière qu'une de ses surfaces soit exposée au fluide et que son autre surface soit en contact avec une couche d'un second électrolyte, lequel est en contact avec une seconde électrode (16) de travail; et

un circuit agencé de manière à maintenir la première électrode de travail à un potentiel auquel l'oxygène est réduit, et comprenant des première et seconde contre-électrodes (28, 30) en contact avec les électrolytes respectifs;

appareil caractérisé en ce que:

la première couche (32) du haut polymère est perméable à la fois au bioxyde de carbone et à l'oxygène et est en contact, par ladite autre surface, avec une couche poreuse de métal (34) qui constitue la première électrode de travail;

la seconde couche (22) de haut polymère est agencée "en série" avec la première couche (32), de sorte que la première surface de la seconde couche est exposée à du bioxyde de carbone dont l'oxygène a été enlevé par passage à travers le premier électrolyte;

le seconde électrolyte est non aqueux; et

le circuit (48) est agencé de manière à maintenir la première électrode de travail à un potentiel tel que l'oxygène, mais non pas le bioxyde de carbone, est réduit dans le premier électrolyte aqueux, de manière à maintenir la seconde électrode de travail à un potentiel tel que le bioxyde de carbone est réduit dans le second électrolyte non aqueux, et à déceler tout courant s'écoulant entre la seconde électrode de travail et la seconde contré-électrode.

2. Appareil selon la revendication 1, caractérisé en ce que la première couche de haut polymère (32) et la première électrode (34) de travail prennent la forme d'une membrane métallisée constituée par la matière du haut polymère.

3. Appareil selon la revendication 1 ou la revendication 2, dans lequel le second électrolyte non aqueux est du diméthylsulfoxyde.

4. Appareil selon l'une quelconque des revendications précédentes, caractérisé en ce qu'il comprend également une première électrode (26) de référence en contact avec le premier électrolyte aqueux; et une seconde électrode (18) de référence en contact avec le second électrolyte non aqueux; et en ce que le circuit (48) comprend un moyen permettant de maintenir le potentiel de chaque électrode de travail constant par rapport à son électrode de référence respective.

5. Appareil selon l'une quelconque des revendications précédentes, dans lequel la première électrode de travail a une surface supérieure à celle de la seconde électrode (16) de travail, et l'appareil comporte en outre une limitation (42) destinée à limiter l'accès du fluide vers la première couche (32) de haut polymère.

6. Procédé pour déceler la présence du bioxyde de carbone dans un fluide pouvant contenir de l'oxygène, comprenant:

l'agencement d'une première couche de haut polymère, matière qui est perméable à l'oxygène, de façon qu'une de ses surfaces soit exposée au fluide et que l'autre surface communique avec une couche d'un premier électrolyte aqueux et avec une première électrode de travail;

l'agencement d'une seconde couche (22) de haut polymère, matière qui est perméable au bioxyde de carbone de manière qu'une de ses surfaces soit exposée au fluide et que son autre surface soit en contact avec une seconde électrode (16) de travail; et

le maintien de la première électrode de travail à un potentiel auquel l'oxygène est réduit;

procédé caractérisé en ce que:

la première couche (32) de haut polymère est perméable à la fois au bioxyde de carbone et à l'oxygène et son autre surface est en contact avec une couche poreuse de métal (34) qui constitue la première électrode de travail;

la seconde couche (32) de haut polymère est une matière agencée "en série" avec la première couche (32), de sorte qu'une surface de la seconde couche est exposée à du bioxyde de carbone dont l'oxygène a été enlevé par passage à travers le premier électrolyte;

le second électrolyte est non aqueux; et

la première électrode de travail est maintenue à un potentiel tel que l'oxygène, mais non pas le bioxyde de carbone, est réduit dans le premier électrolyte aqueux, et tout courant traversant la seconde électrode de travail est décelé.

Fig. 1

Fig. 2

1